# EUROPÄISCHE PATENTSCHRIFT

(11) **EP 1 034 166 B1**
(45) Veröffentlichungstag und Bekanntmachung des Hinweises auf die Patenterteilung: **02.05.2003**
(21) Anmeldenummer: 98945309.7
(22) Anmeldetag: 05.09.1998
(51) Int. Cl.: C07D 237/20, C07D 405/10, A01N 43/58

(54) **SUBSTITUIERTE 2-PHENYL-3(2H)-PYRIDAZINONE**
SUBSTITUTED 2-PHENYL-3(2H)-PYRIDAZINONES
2-PHENYLE-3(2H)-PYRIDAZINONES SUBSTITUEES

(30) Priorität: 17.09.1997 DE 19740973
(43) Veröffentlichungstag der Anmeldung: 13.09.2000
(73) Patentinhaber: BASF AKTIENGESELLSCHAFT, 67056 Ludwigshafen (DE)
(72) Erfinder: SCHÄFER, Peter, D-67308 Ottersheim (DE); HAMPRECHT, Gerhard, D-69469 Weinheim (DE); MENGES, Markus, D-64625 Bensheim (DE); MENKE, Olaf, D-67317 Altleiningen (DE); RACK, Michael, D-69123 Heidelberg (DE); REINHARD, Robert, D-67061 Ludwigshafen (DE); ZAGAR, Cyrill, D-67061 Ludwigshafen (DE); MÜNSTER, Peter, D-67354 Römerberg (DE); WESTPHALEN, Karl-Otto, D-67346 Speyer (DE); OTTEN, Martina, D-67069 Ludwigshafen (DE); WALTER, Helmut, D-67283 Obrigheim (DE)
(86) Internationale Anmeldenummer: EP9805622
(87) Internationale Veröffentlichungsnummer: WO99014201

(56) Entgegenhaltungen:
- WO-A-96/39392
- Y. MAKI ET. AL.: "Studies on the Smiles Rearrangement. XII. Synthesis and Structural Assignment of Two Isomeric N-Phenyl 2,3-diazaphenothiazinones." CHEMICAL AND PHARMACEUTICAL BULLETIN, Bd. 21, Nr. 2, Februar 1973, Seiten 241-7, XP002086877
- M. TAKAYA ET. AL.: "Studies on Pyridazinone Derivatives. IX. Synthesis and Hemostatic Activity of 2,4,5-Trisubstituted 3(2H)-pyridazinones." YAKAGUKU ZASSHI, Bd. 98, 1978, Seite 1530-5 XP002086878

## Beschreibung

Die vorliegende Erfindung betrifft neue substituierte 2-Phenyl-3(2H)-pyridazinone der Formel I 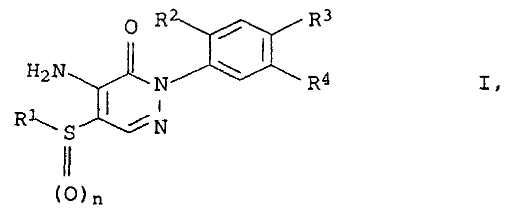 in der die Variablen folgende Bedeutungen haben:
- n: 1 oder 2;
- R¹: C₁-C₄-Alkyl oder C₁-C₄-Halogenalkyl;
- R²: Wasserstoff oder Halogen;
- R³: Halogen oder Cyano;
- R⁴: Wasserstoff, Nitro, Cyano, Formyl, Hydroxylamino, C₁-C₄-Alkyl, C₁-C₄-Halogenalkyl, -OR⁵, -CH=N-OR⁶, -CH=C(R⁷)-CO-OR⁸, -CO-OR⁹, (C₁-C₆-Alkylsulfonyl)amino, Di(C₁-C₆-alkylsulfonyl)amino oder 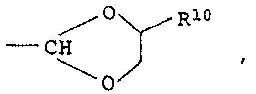 wobei
R⁵ für C₁-C₆-Alkyl, C₃-C₆-Alkenyl, C₃-C₆-Alkinyl, (C₁-C₆-Alkoxy)carbonyl-C₁-C₄-alkyl, (C₃-C₆-Alkenyloxy)carbonyl-C₁-C₄-alkyl, (C₃-C₆-Alkinyloxy)carbonyl-C₁-C₄-alkyl oder C₁-C₄-Alkoxy-(C₁-C₄-alkoxy)carbonyl-C₁-C₄-alkyl,
R⁶ für Wasserstoff, C₁-C₄-Alkyl, Hydroxycarbonyl-C₁-C₄-alkyl oder (C₁-C₄-Alkoxy)carbonyl-C₁-C₄-alkyl,
R⁷ für Wasserstoff, Halogen oder C₁-C₄-Alkyl,
R⁸ für Wasserstoff oder C₁-C₆-Alkyl,
R⁹ für Wasserstoff, C₁-C₆-Alkyl, C₃-C₆-Alkenyl, C₃-C₆-Alkinyl, C₁-C₄-Alkoxy-C₁-C₄-alkoxy, Hydroxycarbonyl-C₁-C₄-alkyl, (C₁-C₆-Alkoxy)carbonyl-C₁-C₄-alkyl, (C₃-C₆-Alkenyloxy)carbonyl-C₁-C₄-alkyl, (C₃-C₆-Alkinyloxy)carbonyl-C₁-C₄-alkyl oder C₁-C₄-Alkoxy-(C₁-C₄-alkoxy)carbonyl-C₁-C₄-alkyl und
R¹⁰ für Wasserstoff, C₁-C₆-Alkyl, Hydroxycarbonyl oder (C₁-C₆-Alkoxy)carbonyl stehen,
sowie die landwirtschaftlich brauchbaren Salze der Verbindungen I.

Außerdem betrifft die Erfindung
- die Verwendung der Verbindungen I als Herbizide oder zur Desikkation/Defoliation von Pflanzen,
- herbizide Mittel und Mittel zur Desikkation/Defoliation von Pflanzen, welche die Verbindungen I als wirksame Substanzen enthalten,
- Verfahren zur Bekämpfung von unerwünschtem Pflanzenwuchs und zur Desikkation/Defoliation von Pflanzen mit den Verbindungen I,
- Verfahren zur Herstellung der Verbindung I und von herbiziden Mitteln und Mitteln zur Desikkation/Defoliation von Pflanzen unter Verwendung der Verbindungen I.

In der WO 96/39392 werden bereits bestimmte Phenylpyridazinone als herbizide Wirkstoffe gelehrt, unter deren allgemeine Formel - bei geeigneter Wahl der Substituenten - formal auch einige der vorliegenden Verbindungen I fallen.

Gegenstand der WO 97/07104 sind u.a. 2-Phenyl-5-haloalkylpyridazin-3-one, denen ebenfalls eine herbizide Wirkung zugeschrieben wird. Im Gegensatz zu den vorliegenden Verbindungen I weisen sie jedoch keine Aminogruppe in 4-Position und keine Schwefelbrücke in 5-Position des Pyridazinon-Ringes auf.

Die herbiziden Eigenschaften der bekannten Verbindungen sind nicht immer völlig befriedigend. Aufgabe der vorliegenden Erfindung war es demnach, neue Phenylpyridazinone mit besseren herbiziden Eigenschaften bereitzustellen, mit denen sich die unerwünschten Pflanzen noch gezielter bekämpfen lassen. Die Aufgabe erstreckt sich auch auf die Bereitstellung neuer desikkant/defoliant wirksamer Verbindungen.

Demgemäß wurden die eingangs definierten substituierten 2-Phenyl-3(2H)-pyridazinone der Formel I gefunden. Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Desikkation und/oder Defoliation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Bei Verbindungen I mit mindestens einem olefinischen Rest sind gegebenenfalls auch E-/Z-Isomere möglich. Gegenstand der Erfindung sind sowohl die reinen Enantiomeren oder Diastereomeren als auch deren Gemische.

Die substituierten 2-Phenyl-3(2H)-pyridanzinone I können in Form ihrer landwirtschaftlich brauchbaren Salze vorliegen, wobei es auf die Art des Salzes in der Regel nicht ankommt. Im allgemeinen kommen die Salze von solchen Basen und diejenigen Säureadditionssalze in Betracht, bei denen die herbizide Wirkung im Vergleich zu der freien Verbindung I nicht negativ beeinträchtigt ist.

Als basische Salze eignen sich besonders diejenigen der Alkalimetalle, vorzugsweise die Natrium- und Kaliumsalze, die der Erdalkalimetalle, vorzugsweise Calcium- und Magnesiumsalze, die der Übergangsmetalle, vorzugsweise Zink- und Eisensalze, sowie Ammoniumsalze, bei denen das Ammoniumion gewünschtenfalls ein bis vier C₁-C₄-Alkyl-, Hydroxy-C₁-C₄-alkylsubstituenten und/oder einen Phenyl- oder Benzylsubstituenten tragen kann, vorzugsweise Diisopropylammonium-, Tetramethylammonium-, Tetrabutylammonium-, Trimethylbenzylammonium- und Trimethyl-(2-hydroxyethyl)-ammoniumsalze, des weiteren Phosphoniumsalze, Sulfoniumsalze wie vorzugsweise Tri-(C₁-C₄-alkyl)sulfoniumsalze, und Sulfoxoniumsalze wie vorzugsweise Tri-(C₁-C₄-alkyl)sulfoxoniumsalze.

Unter den Säureadditionssalzen sind in erster Linie die Hydrochloride und -bromide, Sulfate, Nitrate, Phosphate, Oxalate oder die Dodecylbenzolsulfonate zu nennen.

Die bei der Definition der Substituenten R¹ und R⁴ bis R¹⁰ genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Hydroxycarbonylalkyl-, Alkoxy-, Alkenyl-, Alkenyloxy-, Alkinyl- und Alkinyloxy-Teile können geradkettig oder verzweigt sein. Halogenierte Substituenten tragen vorzugsweise ein bis fünf gleiche oder verschiedene Halogenatome. Die Bedeutung Halogen steht jeweils für Fluor, Chlor, Brom oder Iod.

Ferner stehen beispielsweise:
- C₁-C₄-Alkyl für: CH₃, C₂H₅, CH₂-C₂H₅, CH(CH₃)₂, n-Butyl, CH(CH₃)-C₂H₅, CH₂-CH(CH₃)₂ oder C(CH₃)₃;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. CH₂F, CHF2, CF₃, CH₂Cl, CH(Cl)₂, C(Cl)₃, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, CH₂-C₂F₅, CF₂-C₂F₅, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder n-C₄F₉;
- C₁-C₆-Alkyl für: einen C₁-C₄-Alkyl-Rest wie vorstehend genannt, oder z.B. n-Pentyl, 1-Methylbutyl, 2-Methylbutyl, 3-Methylbutyl, CH₂-C(CH₃)₃, 1-Ethylpropyl, n-Hexyl, C(CH₃)₂-C₂H₅, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, vorzugsweise für CH₃, C₂H₅, CH₂-C₂H₅, CH(CH₃)₂, n-Butyl, C(CH₃)₃, n-Pentyl oder n-Hexyl;
- Hydroxycarbonyl-C₁-C₄-alkyl für: CH₂COOH, 1-(COOH)ethyl, 2-(COOH)ethyl, 1-(COOH)prop-1-yl, 2-(COOH)prop-1-yl, 3-(COOH)prop-1-yl, 1-(COOH)but-1-yl, 2-(COOH)but-1-yl, 3-(COOH)but-1-yl, 4-(COOH)but-1-yl, 1-(COOH)but-2-yl, 2-(COOH)but-2-yl, 3-(COOH)but-2-yl, 4-(COOH)but-2-yl, 1-(CH₂COOH)eth-1-yl, 1-(CH₂COOH)-1-(CH₃)-eth-1-yl oder 1-(CH₂COOH)prop-1-yl;
- (C₁-C₆-Alkoxy)carbonyl für: z.B. CO-OCH₃, CO-OC₂H₅, CO-OCH₂-C₂H₅, CO-OCH(CH₃)₂, CO-O(n-C₄H₉), CO-OCH(CH₃)-C₂H₅, CO-OCH₂-CH(CH₃)₂, CO-OC(CH₃)₃, CO-O(n-C₅H₁₁), 1-Methylbutoxycarbonyl, 2-Methylbutoxycarbonyl, 3-Methylbutoxycarbonyl, CO-OCH₂-C(CH₃)₃, CO-OCH(C₂H₅)-C₂H₅, CO-O(n-C₆H₁₃), CO-OC(CH₃)₂-C₂H₅, CO-OCH(CH₃)-CH(CH₃)₂, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, CO-OC(CH₃)₂-CH(CH₃)₂, CO-OCH(CH₃)-C(CH₃)₃, 1-Ethyl-1-methylpropoxycarbonyl oder CO-OCH(C₂H₅)-CH(CH₃)₂, insbesondere CO-OCH₃, CO-OC₂H₅, CO-OCH₂-C₂H₅ oder CO-OC(CH₃)₂;
- (C₁-C₆-Alkoxy)carbonyl-C₁-C₄-alkyl für: durch (C₁-C₆-Alkoxy)carbonyl wie vorstehend genannt substituiertes C₁-C₄-Alkyl, also z.B. für CH₂COOCH₃, CH₂COOC₂H₅, CH₂COOCH₂-C₂H₅, CH₂COOCH(CH₃)₂, CH₂COOCH₂CH₂-C₂H₅, CH₂COOCH(CH₃)-C₂H₅, CH₂COOCH₂-CH(CH₃)₂, CH₂COOC(CH₃)₃, CH₂COO(CH₂)₃-C₂H₅, CH₂COO(CH₂)₄-C₂H₅, CH(CH₃)COOCH₃, CH(CH₃)COOC₂H₅, CH₂CH₂COOCH₃, CH₂CH₂COOC₂H₅, CH₂CH₂COOCH₂-C₂H₅, CH₂CH₂COOCH(CH₃)₂, CH₂CH₂COOCH₂CH₂-C₂H₅, 2-[COOCH(CH₃)-C₂H₅]ethyl, 2-[COOCH₂-CH(CH₃)₂]ethyl, CH₂CH₂COOC(CH₃)₃, CH₂CH₂COO(CH₂)₃-C₂H₅, CH₂CH₂COO(CH₂)₄-C₂H₅, 2-(COOCH₃)propyl, 2-(COOC₂H₅)propyl, 2-(COOCH₂-C₂H₅)propyl, 2-[COOCH(CH₃)₂]propyl, 2-(COOCH₂CH₂-C₂H₅)propyl, 2-[COOCH(CH₃)-C₂H₅]propyl, 2-[COOCH₂-CH(CH₃)₂]propyl, 2-[COOC(CH₃)₃]propyl, 3-(COOCH₃)propyl, 3-(COOC₂H₅)propyl, 3-(COOCH₂-C₂H₅)propyl, 3-[COOCH(CH₃)₂]propyl, 3-(COOCH₂CH₂-C₂H₅)propyl, 3-[COOCH(CH₃)-C₂H₅]propyl, 3-[COOCH₂-CH(CH₃)₂]propyl, 3-[COOC(CH₃)₃]propyl, 3-[COO(CH₂)₃-C₂H₅]propyl, 3-[COO(CH₂)₄-C₂H₅]propyl, 2-(COOCH₃)butyl, 2-(COOC₂H₅)butyl, 2-(COOCH₂-C₂H₅)butyl, 2-[COOCH(CH₃)₂]butyl, 2-(COOCH₂CH₂-C₂H₅)butyl, 2- [COOCH(CH₃)-C₂H₅]butyl, 2-[COOCH₂-CH(CH₃)₂]butyl, 2-[COOC(CH₃)₃]butyl, 3-(COOCH₃)butyl, 3-(COOC₂H₅)butyl, 3-(COOCH₂-C₂H₅)butyl, 3-[COOCH(CH₃)₂]butyl, 3-(COOCH₂CH₂-C₂H₅)butyl, 3-[COOCH(CH₃)-C₂H₅]butyl, 3-[COOCH₂-CH(CH₃)₂]butyl, 3-[COOC(CH₃)₃]butyl, 4-(COOCH₃)butyl, 4-(COOC₂H₅)butyl, 4-(COOCH₂-C₂H₅)butyl, 4-[COOCH(CH₃)₂]butyl, 4-(COOCH₂CH₂-C₂H₅)butyl, 4-[COOCH(CH₃)-C₂H₅]butyl, 4-[COOCH₂-CH(CH₃)₂]butyl, 4-[COOC(CH₃)₃]butyl, 4-[COO (CH₂)₃-C₂H₅]butyl oder 4-[COO (CH₂)₄-C₂H₅]butyl, insbesondere für CH₂-CO-OCH₃, CH₂-CO-OC₂H₅, CH(CH₃)-CO-OCH₃ oder CH(CH₃)-CO-OC₂H₅;
- C₁-C₄-Alkoxy-(C₁-C₄-alkoxy)carbonyl-C₁-C₄-alkyl für: durch C₁-C₄-Alkoxy wie OCH₃, OC₂H₅, OCH₂-C₂H₅, OCH(CH₃)₂, n-Butoxy, OCH(CH₃)-C₂H₅, OCH₂-CH(CH₃)₂ oder OC(CH₃)₃ - vorzugsweise OCH₃ oder OC₂H₅ - substituiertes (C₁-C₄-Alkoxy)carbonyl-C₁-C₄-alkyl, also z.B. für CH₂-COOCH₂-OCH₃, CH₂-COOCH₂-OC₂H₅, CH₂-COOCH₂-OCH(CH₃)₂, CH₂-COOCH₂-OC(CH₃)₃, CH₂-COOCH₂CH₂-OCH₃, CH₂-COOCH₂CH₂-OC₂H₅, CH(CH₃)-COOCH₂-OCH₃, CH(CH₃)-COOCH₂-OC₂H₅, CH(CH₃)-COOCH₂CH₂-OCH₃ oder CH(CH₃)-COOCH₂CH₂-OC₂H₅, insbesondere für CH₂-COOCH₂CH₂-OCH₃, CH₂-COOCH₂CH₂-OC₂H₅, CH(CH₃)-COOCH₂CH₂-OCH₃ oder CH(CH₃)-COOCH₂CH₂-OC₂H₅;
- (C₁-C₆-Alkylsulfonyl)amino für: NH-SO₂-CH₃, NH-SO₂-C₂H₅, NH-SO₂-(n-C₃H₇), NH-SO₂-CH(CH₃)₂, NH-SO₂-(n-C₄H₉), NH-SO₂-CH(CH₃)-C₂H₅, NH-SO₂-CH₂-CH (CH₃)₂, NH-SO₂-C(CH₃)₃, NH-SO₂-(n-C₅H₁₁), NH-SO₂-CH(CH₃)-CH₂-C₂H₅, NH-SO₂-CH₂-CH(CH₃)-C₂H₅, NH-SO₂-(CH₂)₂-CH(CH₃)₂, NH-SO₂-C(CH₃)₂-C₂H₅, NH-SO₂-CH(CH₃)-CH(CH₃)₂, NH-SO₂-CH₂-C(CH₃)₃, NH-SO₂-CH(C₂H₅)₂, NH-SO₂-(n-C₆H₁₃), NH-SO₂-CH(CH₃)-(n-C₄H₉), NH-SO₂-CH₂-CH(CH₃)-CH₂-C₂H₅, NH-SO₂-(CH₂)₂-CH(CH₃)-C₂H₅, NH-SO₂-(CH₂)₃-CH(CH₃)₂, NH-SO₂-C(CH₃)₂-CH₂-C₂H₅, NH-SO₂-CH(CH₃)-CH(CH₃)-C₂H₅, NH-SO₂-CH(CH₃)-CH₂-CH(CH₃)₂, NH-SO₂-CH₂-C(CH₃)₂-C₂H₅, NH-SO₂-CH₂-CH(CH₃)-CH(CH₃)₂, NH-SO₂-CH₂-CH₂-C(CH₃)₃, NH-SO₂-CH(C₂H₅)-CH₂-C₂H₅, NH-SO₂-CH₂-CH(C₂H₅)₂, NH-SO₂-C(CH₃)₂-CH(CH₃)₂, NH-SO₂-CH(CH₃)-C(CH₃)₃, NH-SO₂-C(C₂H₅)₂-CH₃ oder NH-SO₂-CH(C₂H₅)-CH(CH₃)₂, insbesondere für NH-SO₂-CH₃ oder NH-SO₂-C₂H₅;
- Di(C₁-C₆-alkylsulfonyl)amino für: z.B. N(SO₂-CH₃)₂, N(SO₂-C₂H₅)₂, N(SO₂-CH₂-C₂H₅)₂, N[SO₂-CH(CH₃)₂]₂, N[SO₂-(n-C₄H₉)]₂, N[SO₂-CH(CH₃)-C₂H₅]₂, N[SO₂-CH₂-CH(CH₃)₂]₂, N[SO₂-C(CH₃)₃]₂, N(SO₂-CH₃)-SO₂-C₂H₅, N(SO₂-CH₃)-SO₂-CH₂-C₂H₅, N(SO₂-CH₃)-SO₂-CH(CH₃)₂, N(SO₂-CH₃)-SO₂-(n-C₄H₉), N(SO₂-CH₃)-SO₂-CH(CH₃)-C₂H₅, N(SO₂-CH₃)-SO₂-CH₂-CH(CH₃)₂, N(SO₂-CH₃)-SO₂-C(CH₃)₃, N(SO₂-C₂H₅)-SO₂-CH₂-C₂H₅, N(SO₂-C₂H₅)-SO₂-CH(CH₃)₂, N(SO₂-C₂H₅)-SO₂-(n-C₄H₉), N(SO₂-C₂H₅)-SO₂-CH(CH₃)-C₂H₅, N(SO₂-C₂H₅)-SO₂-CH₂-CH(CH₃)₂, N(SO₂-C₂H₅)-SO₂-C(CH₃)₃, N(SO₂-CH₂-C₂H₅)-SO₂-CH(CH₃)₂, N(SO₂-CH₂-C₂H₅)-SO₂-(n-C₄H₉), N (SO₂-CH₂-C₂H₅)-SO₂-CH(CH₃)-C₂H₅, N(SO₂-CH₂-C₂H₅)-SO₂-CH₂-CH(CH₃)₂, N(SO₂-CH₂-C₂H₅)-SO₂-C(CH₃)₃, N[SO₂-CH(CH₃)₂]-SO₂-(n-C₄H₉), N[SO₂-CH(CH₃)₂]-SO₂-CH(CH₃)-C₂H₅, N[SO₂-CH(CH₃)₂]-SO₂-CH₂-CH(CH₃)₂, N[SO₂-CH(CH₃)₂]-SO₂-C(CH₃)₃, N[SO₂-(n-C₄H₉)]-SO₂-CH(CH₃)-C₂H₅, N[SO₂-(n-C₄H₉)]-SO₂-CH₂-CH(CH₃)₂, N[SO₂-(n-C₄H₉)]-SO₂-C(CH₃)₃, N[SO₂-CH(CH₃)-C₂H₅]-SO₂-CH₂-CH(CH₃)₂, N[SO₂-C(CH₃)₃]-SO₂-CH(CH₃)-C₂H₅ oder N[SO₂-C(CH₃)₃]-SO₂-CH₂-CH(CH₃)₂, insbesondere für N(SO₂-CH₃)₂ oder N(SO₂-C₂H₅);
- C₃-C₆-Alkenyl für: Prop-1-en-1-yl, Allyl, 1-Methylethenyl, 1-Buten-1-yl, 1-Buten-2-yl, 1-Buten-3-yl, 2-Buten-1-yl, 1-Methyl-prop-1-en-1-yl, 2-Methyl-prop-1-en-1-yl; 1-Methylprop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methylbut-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethylprop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-l-yl, 3-Methyl-pent-1-en-1-yl, 4-Methyl-pent-1-en-1-yl, 1-Methylpent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methylpent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethylbut-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethylbut-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- (C₃-C₆-Alkenyloxy)carbonyl-C₁-C₄-alkyl für: durch (C₃-C₆-Alkenyloxy)carbonyl wie Prop-1-en-1-yloxycarbonyl, Prop-2-en-1-yloxycarbonyl, 1-Methylethenyloxycarbonyl, n-Buten-1-yloxycarbonyl, n-Buten-2-yloxycarbonyl, n-Buten-3-yloxycarbonyl, 1-Methyl-prop-1-en-1-yloxycarbonyl, 2-Methylprop-1-en-1-yloxycarbonyl, 1-Methyl-prop-2-en-1-yloxycarbonyl, 2-Methyl-prop-2-en-1-yloxycarbonyl, n-Penten-1-yloxycarbonyl, n-Penten-2-yloxycarbonyl, n-Penten-3-yloxycarbonyl, n-Penten-4-yloxycarbonyl, 1-Methyl-but-1-en-1-yloxycarbonyl, 2-Methylbut-1-en-1-yloxycarbonyl, 3-Methyl-but-1-en-1-yloxycarbonyl, 1-Methyl-but-2-en-1-yloxycarbonyl, 2-Methyl-but-2-en-1-yloxycarbonyl, 3-Methyl-but-2-en-1-yloxycarbonyl, 1-Methylbut-3-en-1-yloxycarbonyl, 2-Methyl-but-3-en-1-yloxycarbonyl, 3-Methyl-but-3-en-1-yloxycarbonyl, 1,1-Dimethylprop-2-en-1-yloxycarbonyl, 1,2-Dimethyl-prop-1-en-1-yloxycarbonyl, 1,2-Dimethyl-prop-2-en-1-yloxycarbonyl, 1-Ethyl-prop-1-en-2-yloxycarbonyl, 1-Ethyl-prop-2-en-l-yloxycarbonyl, n-Hex-1-en-1-yloxycarbonyl, n-Hex-2-en-1-yloxycarbonyl, n-Hex-3-en-1-yloxycarbonyl, n-Hex-4-en-1-yloxycarbonyl, n-Hex-5-en-1-yloxycarbonyl, 1-Methyl-pent-1-en-1-yloxycarbonyl, 2-Methyl-pent-1-en-1-yloxycarbonyl, 3-Methyl-pent-1-en-1-yloxycarbonyl, 4-Methyl-pent-1-en-1-yloxycarbonyl, 1-Methyl-pent-2-en-1-yloxycarbonyl, 2-Methyl-pent-2-en-1-yloxycarbonyl, 3-Methyl-pent-2-en-1-yloxycarbonyl, 4-Methyl-pent-2-en-1-yloxycarbonyl, 1-Methyl-pent-3-en-1-yloxycarbonyl, 2-Methylpent-3-en-1-yloxycarbonyl, 3-Methyl-pent-3-en-1-yloxycarbonyl, 4-Methyl-pent-3-en-1-yloxycarbonyl, 1-Methyl-pent-4-en-1-yloxycarbonyl, 2-Methyl-pent-4-en-1-yloxycarbonyl, 3-Methyl-pent-4-en-1-yloxycarbonyl, 4-Methyl-pent-4-en-1-yloxycarbonyl, 1,1-Dimethyl-but-2-en-1-yloxycarbonyl, 1,1-Dimethyl-but-3-en-1-yloxycarbonyl, 1,2-Dimethyl-but-1-en-1-yloxycarbonyl, 1,2-Dimethylbut-2-en-1-yloxycarbonyl, 1,2-Dimethyl-but-3-en-1-yloxycarbonyl, 1,3-Dimethyl-but-1-en-1-yloxycarbonyl, 1,3-Dimethylbut-2-en-1-yloxycarbonyl, 1,3-Dimethyl-but-3-en-1-yloxycarbonyl, 2,2-Dimethyl-but-3-en-1-yloxycarbonyl, 2,3-Dimethylbut-1-en-1-yloxycarbonyl, 2,3-Dimethyl-but-2-en-1-yloxycarbonyl, 2,3-Dimethyl-but-3-en-1-yloxycarbonyl, 3,3-Dimethylbut-1-en-1-yloxycarbonyl, 3,3-Dimethyl-but-2-en-1-yloxycarbonyl, 1-Ethyl-but-1-en-1-yloxycarbonyl, 1-Ethyl-but-2-en-1-yloxycarbonyl, 1-Ethyl-but-3-en-1-yloxycarbonyl, 2-Ethylbut-1-en-1-yloxycarbonyl, 2-Ethyl-but-2-en-1-yloxycarbonyl, 2-Ethyl-but-3-en-1-yloxycarbonyl, 1,1,2-Trimethyl-prop-2-en-1-yloxycarbonyl, 1-Ethyl-1-methyl-prop-2-en-1-yloxycarbonyl, 1-Ethyl-2-methyl-prop-1-en-1-yloxycarbonyl oder 1-Ethyl-2-methyl-prop-2-en-1-yloxycarbonyl, vorzugsweise Prop-2-en-1-yloxycarbonyl, substituiertes C₁-C₄-Alkyl, also beispielsweise Prop-2-en-1-yl-oxycarbonyl-methyl;
- C₃-C₆-Alkinyl für: z.B. Prop-1-in-1-yl, Propargyl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methylpent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl, insbesondere Propargyl;
- (C₃-C₆-Alkinyloxy) carbonyl-C₁-C₄-alkyl für: durch (C₃-C₆-Alkinyloxy)carbonyl wie Prop-1-in-1-yl-O-CO, Propargyl-O-CO, n-But-1-in-1-yl-O-CO, n-But-1-in-3-yl-O-CO, n-But-1-in-4-yl-O-CO, n-But-2-in-1-yl-O-CO, n-Pent-1-in-1-yl-O-CO, n-Pent-1-in-3-yl-O-CO, n-Pent-1-in-4-yl-O-CO, n-Pent-l-in-5-yl-O-CO, n-Pent-2-in-1-yl-O-CO, n-Pent-2-in-4-yl-O-CO, n-Pent-2-in-5-yl-O-CO, 3-Methylbut-1-in-3-yl-O-CO, 3-Methylbut-1-in-4-yl-O-CO, n-Hex-1-in-1-yl-O-CO, n-Hex-1-in-3-yl-O-CO, n-Hex-1-in-4-yl-O-CO, n-Hex-1-in-5-yl-O-CO, n-Hex-l-in-6-yl-O-CO, n-Hex-2-in-1-yl-O-CO, n-Hex-2-in-4-yl-O-CO, n-Hex-2-in-5-yl-O-CO, n-Hex-2-in-6-yl-O-CO, n-Hex-3-in-1-yl-O-CO, n-Hex-3-in-2-yl-O-CO, 3-Methylpent-1-in-1-yl-O-CO, 3-Methylpent-1-in-3-yl-O-CO, 3-Methylpent-1-in-4-yl-O-CO, 3-Methylpent-1-in-5-yl-O-CO, 4-Methylpent-1-in-1-yl-O-CO, 4-Methylpent-2-in-4-yl-O-CO oder 4-Methylpent-2-in-5-yl-O-CO - vorzugsweise Propargyl-O-CO, But-1-in-3-yl-O-CO, But-1-in-4-yl-O-CO oder But-2-in-1-yl-O-CO - substituiertes C₁-C₄-Alkyl,
   also beispielsweise für Propargyl-O-CO-CH₂ oder 2-(Propargyl-O-CO)ethyl.

Im Hinblick auf die Verwendung der erfindungsgemäßen substituierten 2-Phenyl-3(2H)-pyridazinone der Formel I als Herbizide und/oder als defoliant/desikkant wirksame Verbindungen haben die Variablen vorzugsweise folgende Bedeutungen, und zwar jeweils für sich allein oder in Kombination:
- n: 1 oder 2;
- R¹: C₁-C₄-Alkyl;
- R²: Halogen;
- R³: Halogen;
- R⁴: Wasserstoff, Formyl, C₁-C₄-Alkyl, C₁-C₄-Halogenalkyl, -OR⁵, -CH=N-OR⁶, -CO-OR⁹ oder 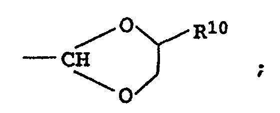
- R⁵: C₁-C₆-Alkyl, C₃-C₆-Alkenyl, C₃-C₆-Alkinyl oder (C₁-C₆-Alkoxy)carbonyl-C₁-C₄-alkyl;
- R⁶: Wasserstoff oder C₁-C₄-Alkyl;
- R⁹: Wasserstoff, C₁-C₆-Alkyl, C₃-C₆-Alkenyl, C₃-C₆-Alkinyl, C₁-C₄-Alkoxy-C₁-C₄-alkoxy oder (C₁-C₆-Alkoxy)carbonyl-C₁-C₄-alkyl;
- R¹⁰: Wasserstoff oder C₁-C₆-Alkyl.

Ganz besonders bevorzugt sind die substituierten 2-Phenyl-3(2H)-pyridazinone der Formel Ia { 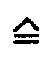 I mit n = 2, R¹ = Methyl, R² = Fluor und R³ = Chlor} 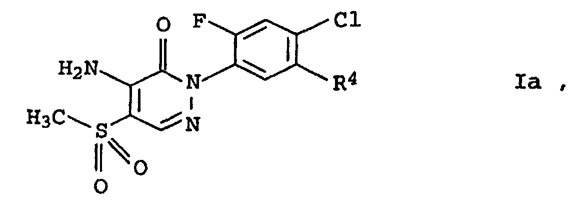 insbesondere die in der folgenden Tabelle 1 aufgeführten Verbindungen Ia.1 bis Ia.141:

**Tabelle 1**

| Nr. | R⁴ |
|---|---|
| Ia.1 | H |
| Ia.2 | OH |
| Ia.3 | OCH₃ |
| Ia.4 | OC₂H₅ |
| Ia.5 | OCH₂-C₂H₅ |
| Ia.6 | OCH(CH₃)₂ |
| Ia.7 | OCH₂-CH₂-C₂H₅ |
| Ia.8 | OCH₂-CH(CH₃)₂ |
| Ia.9 | OCH(CH₃)-C₂H₅ |
| Ia.10 | OC(CH₃)₃ |
| Ia.11 | OCH₂-CH=CH₂ |
| Ia.12 | OCH₂-CH=CH-CH₃ |
| Ia.13 | OCH₂-C(CH₃)=CH₂ |
| Ia.14 | OCH(CH₃)-CH=CH₂ |
| Ia.15 | OCH₂-C≡CH |
| Ia.16 | OCH₂-C≡C-CH₃ |
| Ia.17 | OCH(CH₃)-C≡CH |
| Ia.18 | OCH₂-CO-OCH₃ |
| Ia.19 | OCH₂-CO-OC₂H₅ |
| Ia.20 | OCH₂-CO-OCH₂-C₂H₅ |
| Ia.21 | OCH₂-CO-OCH(CH₃)₂ |
| Ia.22 | OCH₂-CO-OCH₂-CH₂-C₂H₅ |
| Ia.23 | OCH₂-CO-OCH₂-CH(CH₃)₂ |
| Ia.24 | OCH₂-CO-OCH(CH₃)-C₂H₅ |
| Ia.25 | OCH₂-CO-OC(CH₃)₃ |
| Ia.26 | OCH(CH₃)-CO-OCH₃ |
| Ia.27 | OCH(CH₃)-CO-OC₂H₅ |
| Ia.28 | OCH(CH₃)-CO-OCH₂-C₂H₅ |
| Ia.29 | OCH(CH₃)-CO-OCH(CH₃)₂ |
| Ia.30 | OCH(CH₃)-CO-OCH₂-CH₂-C₂H₅ |
| Ia.31 | OCH(CH₃)-CO-OCH₂-CH(CH₃)₂ |
| Ia.32 | OCH(CH₃)-CO-OCH(CH₃)-C₂H₅ |
| Ia.33 | OCH(CH₃)-CO-OC(CH₃)₃ |
| Ia.34 | OCH₂-CO-OCH₂-CH=CH₂ |
| Ia.35 | OCH₂-CO-OCH₂-CH=CH-CH₃ |
| Ia.36 | OCH₂-CO-OCH₂-C(CH₃)=CH₂ |
| Ia.37 | OCH₂-CO-OCH(CH₃)-CH=CH₂ |
| Ia.38 | OCH(CH₃)-CO-OCH₂-CH=CH₂ |
| Ia.39 | OCH(CH₃)-CO-OCH₂-CH=CH-CH₃ |
| Ia.40 | OCH(CH₃)-CO-OCH₂-C(CH₃)=CH₂ |
| Ia.41 | OCH(CH₃)-CO-OCH(CH₃)-CH=CH₂ |
| Ia.42 | OCH₂-CO-OCH₂-C≡C-H |
| Ia.43 | OCH₂-CO-OCH₂-C≡C-CH₃ |
| Ia.44 | OCH₂-CO-OCH(CH₃)-C≡C-H |
| Ia.45 | OCH(CH₃)-CO-OCH₂-C≡C-H |
| Ia.46 | OCH(CH₃)-CO-OCH₂-C≡C-CH₃ |
| Ia.47 | OCH(CH₃)-CO-OCH(CH₃)-C≡C-H |
| Ia.48 | OCH₂-CO-OCH₂-CH₂-OCH₃ |
| Ia.49 | OCH₂-CO-OCH₂-CH₂-OC₂H₅ |
| Ia.50 | OCH(CH₃)-CO-OCH₂-CH₂-OCH₃ |
| Ia.51 | OCH(CH₃)-CO-OCH₂-CH₂-OC₂H₅ |
| Ia.52 | CH₃ |
| Ia.53 | CH₂Cl |
| Ia.54 | CH(Cl)₂ |
| Ia.55 | C(Cl)₃ |
| Ia.56 | CH₂Br |
| Ia.57 | CH(Br)₂ |
| Ia.58 | CHO |
| Ia.59 | CH=N-OH |
| Ia.60 | CH=N-OCH₃ |
| Ia.61 | CH=N-OC₂H₅ |
| Ia.62 | CH=N-OCH₂-C₂H₅ |
| Ia.63 | CH=N-OCH(CH₃)₂ |
| Ia.64 | CH=CH-COOH |
| Ia.65 | CH=CH-CO-OCH₃ |
| Ia.66 | CH=CH-CO-OC₂H₅ |
| Ia.67 | CH=CH-CO-OCH₂-C₂H₅ |
| Ia.68 | CH=CH-CO-OCH(CH₃)₂ |
| Ia.69 | CH=C(CH₃)-COOH |
| Ia.70 | CH=C(CH₃)-CO-OCH₃ |
| Ia.71 | CH=C(CH₃)-CO-OC₂H₅ |
| Ia.72 | CH=C(CH₃)-CO-OCH₂-C₂H₅ |
| Ia.73 | CH=C(CH₃)-CO-OCH(CH₃)₂ |
| Ia.74 | CH=C(Cl)-COOH |
| Ia.75 | CH=C(Cl)-CO-OCH₃ |
| Ia.76 | CH=C(Cl)-CO-OC₂H₅ |
| Ia.77 | CH=C(Cl)-CO-OCH₂-C₂H₅ |
| Ia.78 | CH=C(Cl)-CO-OCH(CH₃)₂ |
| Ia.79 | CN |
| Ia.80 | COOH |
| Ia.81 | CO-OCH₃ |
| Ia.82 | CO-OC₂H₅ |
| Ia.83 | CO-OCH₂-C₂H₅ |
| Ia.84 | CO-OCH(CH₃)₂ |
| Ia.85 | CO-OCH₂-CH₂-C₂H₅ |
| Ia.86 | CO-OCH₂-CH(CH₃)₂ |
| Ia.87 | CO-OCH(CH₃)-C₂H₅ |
| Ia.88 | CO-OC(CH₃)₃ |
| Ia.89 | CO-OCH₂-CH=CH₂ |
| Ia.90 | CO-OCH₂-CH=CH-CH₃ |
| Ia.91 | CO-OCH₂-C(CH₃)=CH₂ |
| Ia.92 | CO-OCH(CH₃)-CH=CH₂ |
| Ia.93 | CO-OCH₂-C≡CH |
| Ia.94 | CO-OCH₂-C≡C-CH₃ |
| Ia.95 | CO-OCH(CH₃)-C≡CH |
| Ia.96 | CO-OCH₂-CH₂-OCH₃ |
| Ia.97 | CO-OCH₂-CH₂-OC₂H₅ |
| Ia.98 | CO-OCH₂-COOH |
| Ia.99 | CO-OCH(CH₃)-COOH |
| Ia.100 | CO-OCH₂-CO-OCH₃ |
| Ia.101 | CO-OCH₂-CO-OC₂H₅ |
| Ia.102 | CO-OCH₂-CO-OCH₂-C₂H₅ |
| Ia.103 | CO-OCH₂-CO-OCH(CH₃)₂ |
| Ia.104 | CO-OCH₂-CO-OCH₂-CH₂-C₂H₅ |
| Ia.105 | CO-OCH₂-CO-OCH₂-CH(CH₃)₂ |
| Ia.106 | CO-OCH₂-CO-OCH(CH₃)-C₂H₅ |
| Ia.107 | CO-OCH₂-CO-OC(CH₃)₃ |
| Ia.108 | CO-OCH(CH₃)-CO-OCH₃ |
| Ia.109 | CO-OCH(CH₃)-CO-OC₂H₅ |
| Ia.110 | CO-OCH(CH₃)-CO-OCH₂-C₂H₅ |
| Ia.111 | CO-OCH(CH₃)-CO-OCH(CH₃)₂ |
| Ia.112 | CO-OCH(CH₃)-CO-OCH₂-CH₂-C₂H₅ |
| Ia.113 | CO-OCH(CH₃)-CO-OCH₂-CH(CH₃)₂ |
| Ia.114 | CO-OCH(CH₃)-CO-OCH(CH₃)-C₂H₅ |
| Ia.115 | CO-OCH(CH₃)-CO-OC(CH₃)₃ |
| Ia.116 | CO-OCH₂-CO-OCH₂-CH=CH₂ |
| Ia.117 | CO-OCH₂-CO-OCH₂-CH=CH-CH₃ |
| Ia.118 | CO-OCH₂-CO-OCH₂-C(CH₃)=CH₂ |
| Ia.119 | CO-OCH₂-CO-OCH(CH₃)-CH=CH₂ |
| Ia.120 | CO-OCH(CH₃)-CO-OCH₂-CH=CH₂ |
| Ia.121 | CO-OCH(CH₃)-CO-OCH₂-CH=CH-CH₃ |
| Ia.122 | CO-OCH(CH₃)-CO-OCH₂-C(CH₃)=CH₂ |
| Ia.123 | CO-OCH(CH₃)-CO-OCH(CH₃)-CH=CH₂ |
| Ia.124 | CO-OCH₂-CO-OCH₂-C≡CH |
| Ia.125 | CO-OCH₂-CO-OCH₂-C≡C-CH₃ |
| Ia.126 | CO-OCH₂-CO-OCH(CH₃)-C≡CH |
| Ia.127 | CO-OCH(CH₃)-CO-OCH₂-C≡CH |
| Ia.128 | CO-OCH(CH₃)-CO-OCH₂-C≡C-CH₃ |
| Ia.129 | CO-OCH(CH₃)-CO-OCH(CH₃)-C≡CH |
| Ia.130 | CO-OCH₂-CO-OCH₂-CH₂-OCH₃ |
| Ia.131 | CO-OCH₂-CO-OCH₂-CH₂-OC₂H₅ |
| Ia.132 | CO-OCH(CH₃)-CO-OCH₂-CH₂-OCH₃ |
| Ia.133 | CO-OCH(CH₃)-CO-OCH₂-CH₂-OC₂H₅ |
| Ia.134 | 1,3-Dioxolan-2-yl |
| Ia.135 | 4-CH₃-1,3-Dioxolan-2-yl |
| Ia.136 | 4-C₂H₅-1,3-Dioxolan-2-yl |
| Ia.137 | 4-(COOH)-1,3-Dioxolan-2-yl |
| Ia.138 | 4-(CO-OCH₃)-1,3-Dioxolan-2-yl |
| Ia.139 | 4-(CO-OC₂H₅)-1,3-Dioxolan-2-yl |
| Ia.140 | 4-(CO-OCH₂-C₂H₅)-1,3-Dioxolan-2-yl |
| Ia.141 | CH=C(Br)-CO-OCH₃ |

Des weiteren sind die substituierten 2-Phenyl-3(2H)-pyridazinone der Formeln Ib bis If besonders bevorzugt, insbesondere
- die Verbindungen Ib.1-Ib.141, die sich von den entsprechenden Verbindungen Ia.1-Ia.141 lediglich dadurch unterscheiden, daß n für 1 steht: 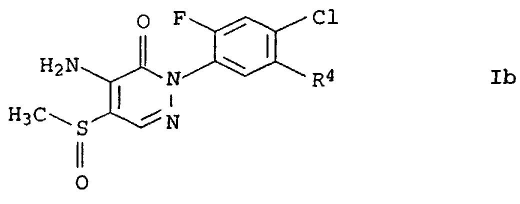
- die Verbindungen Id.1-Id.141, die sich von den entsprechenden Verbindungen Ia.1-Ia.141 lediglich dadurch unterscheiden, daß R³ für Cyano steht: 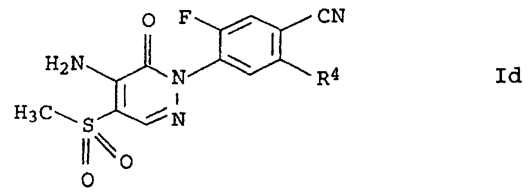
- die Verbindungen Ie.1-Ie.141, die sich von den entsprechenden Verbindungen Ia.1-Ia.141 lediglich dadurch unterscheiden, daß n für 1 und R³ für Cyano stehen:

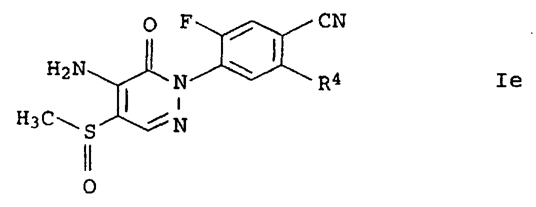

Die substituierten 2-Phenyl-3(2H)-pyridazinone der Formel I sind auf verschiedene Weise erhältlich, insbesondere auf die in folgendem Syntheseschema dargestellte Weise:

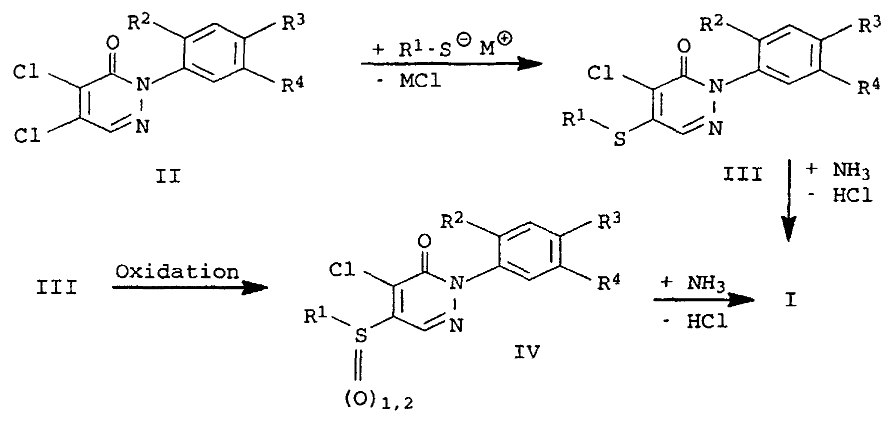

### Syntheseschritt 1

ist die selektive Substitution des Chlors in der 5-Position eines 4,5-Dichlor-3(2H)-pyridazinons (II) durch einen Alkylthiorest. Reaktionen dieser Art sind allgemein bekannt, beispielsweise aus der folgenden Literatur:
K. Dury, Angew. Chem. 77, 282 (1965);
M. Takaya et al., Yakugaku Zasshi 98, 1530 (1978);
J.W. Lyga, J. Het. Chem. 25, 1557 (1988).

Um eine möglichst hohe Selektivität zu erreichen, arbeitet man vorzugsweise in einem polaren Lösungsmittel oder Lösungsmittelgemisch mit hoher Dielektrizitätskonstante (vgl. J.W. Lyga, 1988), beispielsweise in einem niederen Alkohol wie Methanol und Ethanol, oder in N,N-Dimethylformamid, Dimethylsulfoxid, Tetrahydrofuran/Wasser, Pyridin/Wasser oder Methanol/Wasser.

Die Reaktionstemperatur liegt normalerweise bei (-10) bis 50°C, vorzugsweise bei 0 bis 30°C.

Zur Erzielung einer möglichst hohen Selektivität werden II und R¹-S^{⊖} in etwa stöchiometrischen Mengen eingesetzt, oder eine der Komponenten im geringen Überschuß bis etwa 5 mol-%.

Das Verfahrensprodukt III kann gewünschtenfalls am Schwefel zum Sulfoxid oder Sulfon oxidiert werden. Reaktionen dieser Art sind allgemein bekannt, beispielsweise aus der folgenden Literatur:
G. Kresze in Houben Weyl, Methoden der Organischen Chemie, Bd. E11, 1985, S. 669 ff.;
K. Schank in Houben Weyl, Methoden der Organischen Chemie, Bd. E11, S. 1129 ff.;
M. Takaya et al., Yakugaku Zasshi 98, 1530 (1978).

Geeignete Oxidationsmittel sind beispielsweise Peressigsäure, Trifluorperessigsäure, Perbenzoesäure, m-Chlorperbenzoesäure, Monopermaleinsäure, Magnesiummonoperphthalat, Natriumperborat, Oxone® (enthält Peroxidisulfat), Perwolframsäure, Wasserstoffperoxid, Sauerstoff und tert.-Butylhydroperoxid.

Geeignete Lösungsmittel sind z.B. Wasser, Schwefelsäure, Essigsäure, Trifluoressigsäure und halogenierte Kohlenwasserstoffe wie Dichlormethan und Chloroform.

Normalerweise gelingt die Oxidation bei Temperaturen von 0 bis 100°C.

Soll die Oxidation auf der Stufe des Sulfoxids angehalten werden, so arbeitet man vorzugsweise mit etwa äquimolaren Mengen des Oxidationsmittels. Ist die Oxidation zum Sulfon erwünscht, so werden mindestens zwei Äquivalente des Oxidationsmittels, vorzugsweise ein noch größerer Überschuß, bezogen auf die Menge an III, eingesetzt.
Bei den 4-Chlor-5-(halogen)alkylthio-2-phenyl-3(2H)-pyridazinonen III oder deren Oxidationsprodukten IV läßt sich das Chlor in 4-Position am Pyridazinon-Ring durch Reaktion mit Ammoniak auf an sich bekannte Weise substituieren. Beispielhaft sei hierzu auf
M. Takaya et al., Yakugaku Zasshi 98, 1530 (1978) sowie
V. Konecny et al., Coll. Czech. Chem. Commun. 50, 492 (1985) verwiesen.
Die Reaktion kann in einer Vielzahl von Lösungsmitteln erfolgreich durchgeführt werden, beispielsweise in einem Ether wie tert.-Butylmethylether und Tetrahydrofuran, einem aromatischen Kohlenwasserstoff wie Toluol, einem niederen Alkohol wie Methanol und Ethanol oder in einem aprotischen Solvens wie Acetonitril und N,N-Dimethylformamid.
Geht man von III aus, so liegt die Reaktionstemperatur üblicherweise bei 20 bis 150°C; bei der Umsetzung von IV arbeitet man normalerweise bei (-10) bis 100°C, vorzugsweise 0 bis 50°C.
Zur Erzielung einer ausreichend hohen Reaktionsgeschwindigkeit wird der Ammoniak vorzugsweise im großen Überschuß, bezogen auf die Menge an III, eingesetzt. Geht man von IV aus, so ist in der Regel eine stöchiometrische Menge Ammoniak ausreichend; mit überschüssigem Ammoniak kann jedoch häufig eine verbesserte Ausbeute erzielt werden.

Normalerweise sind die substituierten 2-Phenyl-3(2H)-pyridazinone I nach dem vorstehend genannten Syntheseverfahren herstellbar. Aus wirtschaftlichen oder verfahrenstechnischen Gründen kann es jedoch zweckmäßiger sein, einige Verbindungen I aus ähnlichen 2-Phenyl-3(2H)-pyridazinonen, die sich jedoch in der Bedeutung eines Restes unterscheiden, herzustellen.

Die Ausgangsverbindungen II sind bekannt oder in Analogie zu einem in der WO 96/39392 beschriebenen Verfahren erhältlich.

Die Aufarbeitung der Reaktionsgemische erfolgt in der Regel nach an sich bekannten Methoden, beispielsweise
durch Verdünnen der Reaktionslösung mit Wasser und anschließender Isolierung des Produktes mittels Filtration, Kristallisation oder Lösungsmittelextraktion, oder
durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten 2-Phenyl-3(2H)-pyridazinone I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die weitgehend reinen Isomeren getrennt werden können. Reine optisch aktive Isomere lassen sich vorteilhaft aus entsprechenden optisch aktiven Ausgangsprodukten herstellen.

Landwirtschaftlich brauchbare Salze der Verbindungen I können durch Reaktion mit einer Base des entsprechenden Kations, vorzugsweise einem Alkalimetallhydroxid oder -hydrid, gebildet werden.

Salze von I, deren Metallion kein Alkalimetallion ist, können auch durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die substituierten 2-Phenyl-3(2H)-pyridazinone I auch zur Desikkation und/oder Defoliation von Pflanzen.

5 Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark-polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, 5 die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. Ia.2 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. Ia.15 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. Ia.60 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. Ia.71 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. Ia.81 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. Ia.134 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. Ia.135 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 1 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl; BASF AG) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die substituierten 2-Phenyl-3(2H)-pyridazinone I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### (Die chemische Verschiebung [in ppm] der Kernresonanzspektren wurde gemessen gegen Tetramethylsilan)

### Beispiel 1

### 4-Amino-2-(4-chlor-2-fluor-5-propargyloxyphenyl)-5-methylsulfonyl-3(2H)-pyridazinon (Verb. Nr. Ia.15)

Zu einer Lösung von 2,0 g 4-Chlor-2-(4-chlor-2-fluor-5-propargyloxyphenyl)-5-methylsulfonyl-3(2H)-pyridazinon in einer Mischung aus 200 ml Methanol und 30 ml Tetrahydrofuran wurden 10 ml einer 2 molaren Lösung von Ammoniak in Methanol getropft. Nach drei Stunden Rühren engte man ein. Der Rückstand wurde mit tert.-Butyl-methylether verrührt und mittels Chromatographie an Kieselgel (Dichlormethan als Elutionsmittel) weiter gereinigt.
Ausbeute: 1,27 g (weiße Kristalle); Schmp. 168 bis 169°C.
¹H-NMR (400 MHz, in d⁶-Dimethylsulfoxid): δ [ppm] = 3,33 (s,3H), 3,63 (s,1H 4,94 (s,2H), 7,48 (d,1H) 7,62 (br.,1H,N-H), 7,75 (d,1H), 7,97 (s,1H), 8,10 (br.,1H,N-H).

### Vorstufe 1.1: 4,5-Dichlor-2-(4-chlor-2-fluor-5-methoxyphenyl)-3(2H)-pyridazinon

26,6 g Mucochlorsäure und 30,0 g 4-Chlor-2-fluor-5-methoxyphenyl-5 hydrazin wurden in 300 ml Eisessig drei Stunden auf Rückflußtemperatur erhitzt. Anschließend ließ man auf Raumtemperatur abkühlen. Dann wurde der entstandene Feststoff (27 g) abgetrennt, mit Wasser gewaschen und unter reduziertem Druck bei 50°C getrocknet. Die Mutterlösung engte man zunächst weiter ein, bevor 100 ml Wasser zugegeben wurden. Man erhielt so eine zweite Kristallfraktion, die abgetrennt und mit Wasser gewaschen wurde. Nach Verrühren mit Diethylether und Trocknen erhielt man weitere 16,0 g des gewünschten Vorproduktes.
Gesamtausbeute: 43 g (weiße Kristalle); Schmp. 150°C.

### Vorstufe 1.2: 4,5-Dichlor-2-(4-chlor-2-fluor-5-hydroxyphenyl)-3(2H)-pyridazinon und 5-Brom-4-chlor-2-(4-chlor-2-fluor-5-hydroxyphenyl)-3(2H) -pyridazinon

Zu einer Lösung von 36,1 g des Verfahrensproduktes aus Vorstufe 1.1 in 300 ml wasserfreiem Dichlormethan wurden bei 0°C 145 ml einer 1-molaren Lösung von Bortribromid in Dichlormethan getropft. Nach beendeter Zugabe entfernte man die Kühlung und rührte 16 Stunden bei 23°C. Anschließend wurden weitere 32 ml der Bortribromid-Lösung zugetropft, wonach man noch zwei Stunden rührte. Dann wurden unter Eiskühlung 150 ml 10 %iger Salzsäure in die Reaktionsmischung getropft, wobei das Produkt auskristallisierte. Der Feststoff wurde abgetrennt, mit Wasser gewaschen und unter reduziertem Druck bei 50°C getrocknet. Ausbeute: 36,8 g (weiße Kristalle) eines ca. 5:1 Gemisches aus dem 4,5-Dichlorund dem 5-Brom-4-chlor-Derivat.
¹H-NMR (250 MHz, in d⁶-Dimethylsulfoxid): δ [ppm] = 7,14 (d,1H), 7,62 (d,1H), 8,37 (s,1H, 4,5-Dichlor-Derivat), 8,40 (s,1H, 5-Brom-4-chlor-Derivat), 10,70 (br., 1H).

### Vorstufe 1.3: 4,5-Dichlor-2-(4-chlor-2-fluor-5-propargyloxyphenyl)-3(2H)-pyridazinon und 5-Brom-4-chlor-2-(4-chlor-2-fluor-5-propargyloxyphenyl)-3(2H)-pyridazinon

3,6 g einer 80 gew.-%igen Suspension von Natriumhydrid in Mineralöl wurden in 50 ml wasserfreiem Dimethylformamid suspendiert. In die Mischung tropfte man eine Lösung von 38,9 g des Reaktionsprodukts aus Vorstufe 1.2 in 200 ml Dimethylformamid. Nach 15 Minuten Rühren wurden 14,4 g Propargylbromid zugetropft, wonach man 20 Stunden bei 23°C rührte. Dann wurde das Reaktionsgemisch in 1,2 1 Eiswasser gegossen. Anschließend extrahierte man dreimal mit je 200 ml Essigsäureethylester. Die vereinigten organischen Phasen wurden zweimal mit je 50 ml Wasser gewaschen, über Natriumsulfat getrocknet und schließlich eingeengt. Die Reinigung des Rohproduktes erfolgte mittels Chromatographie an Kieselgel (Laufmittel: Cyclohexan/Essigester = 4:1). Ausbeute: 11,1 g (weiße Kristalle) eines ca. 5:1 Gemisches aus dem 4,5-Dichlorund dem 5-Brom-4-chlor-Derivat.
¹H-NMR (250 MHz, in CDCl₃): δ [ppm] = 2,58 (t,1H), 4,78 (d,2H), 7,15 (d,1H) 7,33 (d,1H), 7,94 (s,1H 4,5-Dichlor-Derivat), 8,00 (s,1H, 5-Brom-4-chlor-Derivat).

### Vorstufe 1.4: 4-Chlor-2-(4-chlor-2-fluor-5-propargyloxyphenyl)-5-methylthio-3(2H)-pyridazinon

Zu einer Lösung von 10,9 g des Reaktionsproduktes aus Vorstufe 1.3 in 160 ml Tetrahydrofuran wurde unter Eiskühlung eine Lösung von 2,2 g Natriumthiomethylat in 40 ml Wasser getropft. Nach einer Stunde Rühren bei 0 bis 5°C dampfte man das Tetrahydrofuran ab. Der Feststoffanteil wurde abgetrennt, mit Wasser gewaschen und mit tert.-Butyl-methylether verrührt.
Ausbeute: 9,5 g (weiße Kristalle).
¹H-NMR (270 MHz, in CDCl₃) : δ [ppm] = 2,57 (t,1H), 2,64 (s,3H), 4,77 (d,2H), 7,16 (d,1H), 7,33 (d,1H), 7,81 (s,1H).

### Vorstufe 1.5: 4-Chlor-2-(4-chlor-2-fluor-5-propargyloxyphenyl)-5-methylsulfonyl-3(2H)-pyridazinon

Zu einer Lösung von 9,5 g des Reaktionsproduktes aus Vorstufe 1.4 in 350 ml Chloroform wurden portionsweise 19,6 g m-Chlorperbenzoesäure (56 bis 87 %ig; von Aldrich) gegeben. Anschließend rührte man drei Stunden bei Rückflußtemperatur. Dann wurde die Reaktionsmischung nacheinander mit 10 %iger Natriumhydrogencarbonat-Lösung, zweimal mit 35 %iger Natriumhydrogensulfat-Lösung und zweimal mit Wasser gewaschen. Nach Trocknen über Natriumsulfat engte man ein. Die Reinigung des Rohproduktes erfolgte durch Verrühren mit Diethylether, Abtrennen des ungelösten Anteils und Trocknen unter reduziertem Druck bei 50°C. Ausbeute: 6,7 g (weiße Kristalle); Schmp. 177 bis 178°C.

Auf analoge Weise wurden die folgenden substituierten 2-Phenyl-3(2H)-pyridazinone I hergestellt:

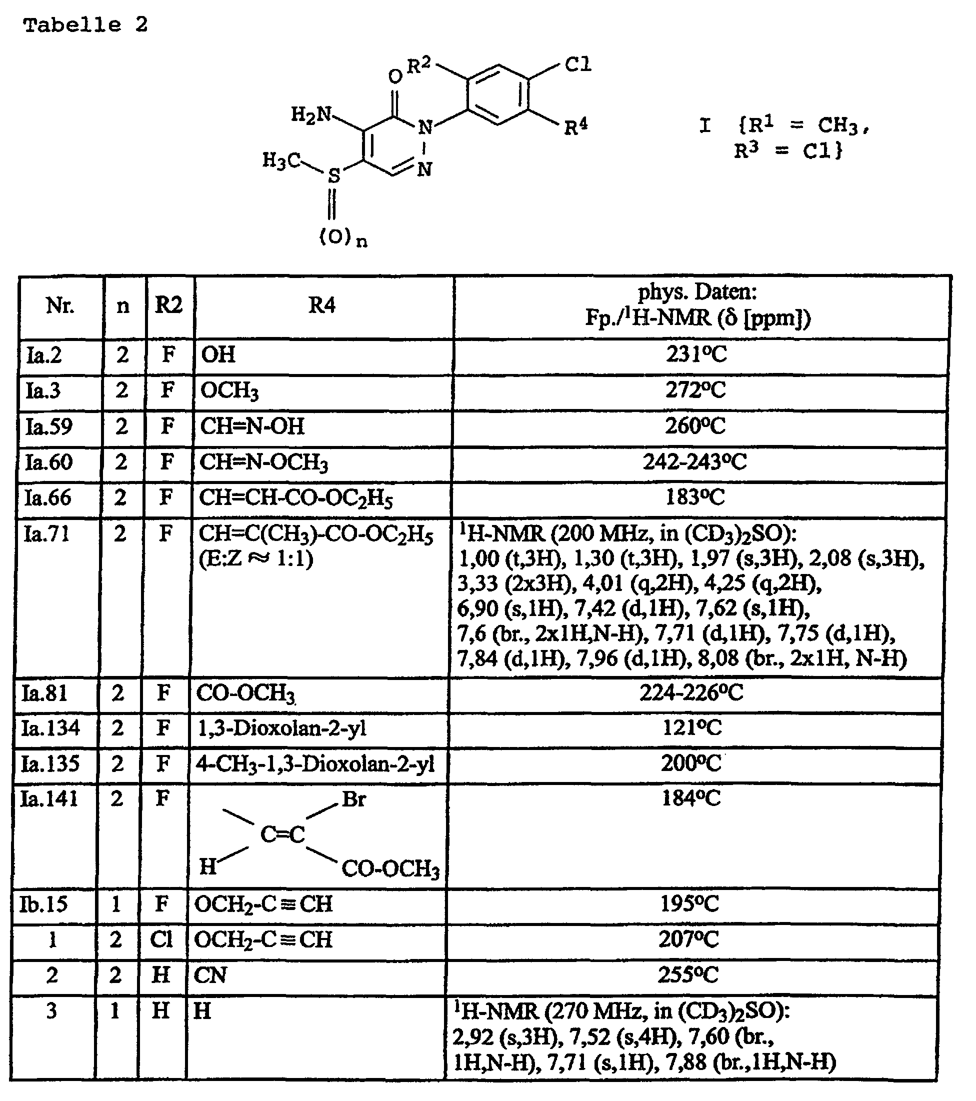

### Anwendungsbeispiele (herbizide Wirksamkeit)

Die herbizide Wirkung der substituierten 2-Phenyl-3(2H)-pyridazinone I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauf laufbehandlung betrug 0,5 oder 0,25 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus | Zurückgekrümmter Fuchsschwanz | redroot pigweed |
| Echinochloa crus-galli | Hühnerhirse | bamyardgrass |
| Ipomoea species | Prunkwindearten | morningglory |
| Setaria viridis | Grüne Borstenhirse | green foxtail |

Bei Aufwandmengen von 0,5 und 0,25 kg/ha a.S. zeigte die Verbindung Nr. Ia.15 im Nachauflaufverfahren eine sehr gute herbizide Wirkung gegen die o.g. Pflanzen.

### Anwendungsbeispiele (desikkant/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wässrigen Aufbereitungen der Wirkstoffe I (unter Zusatz von 0,15 Gew.-% des Fettalkoholalkoxylats Plurafac® LF 700¹⁾, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.
¹⁾ ein schaumarmes, nichtionisches Tensid der BASF AG

Bei den unbehandelten Kontrollpflanzen trat kein Blattfall auf.

## Patentansprüche

1. Substituierte 2-Phenyl-3(2H)-pyridazinone der allgemeinen Formel I 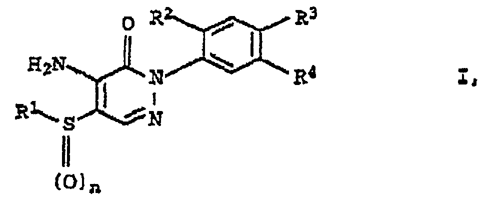 in der die Variablen folgende Bedeutungen haben:
n 1 oder 2;
R¹ C₁-C₄-Alkyl oder C₁-C₄-Halogenalkyl;
R² Wasserstoff oder Halogen;
R³ Halogen oder Cyano;
R⁴ Wasserstoff, Nitro, Cyano, Formyl, Hydroxylamino, C₁-C₄-Alkyl, C₁-C₄-Halogenalkyl, -OR⁵, -CH=N-OR⁶, -CH=C(R⁷)-CO-OR⁸, -CO-OR⁹, (C₁-C₆-Alkylsulfonyl)amino, Di(C₁-C₆-alkylsulfonyl)amino oder 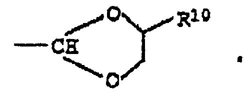 wobei
R⁵ für C₁-C₆-Alkyl, C₃-C₆-Alkenyl, C₃-C₆-Alkinyl, (C₁-C₆-Alkoxy)carbonyl-C₁-C₄-alkyl, (C₃-C₆-Alkenyloxy)carbonyl-C₁-C₄-alkyl, (C₃-C₆-Alkinyloxy)carbonyl-C₁-C₄-alkyl oder C₁-C₄-Alkoxy-(C₁-C₄-alkoxy)carbonyl-C₁-C₄-alkyl,
R⁶ für Wasserstoff, C₁-C₄-Alkyl, Hydroxycarbonyl-C₁-C₄-alkyl oder (C₁-C₄-Alkoxy)carbonyl-C₁-C₄-alkyl,
R⁷ für Wasserstoff, Halogen oder C₁-C₄-Alkyl,
R⁸ für Wasserstoff oder C₁-C₆-Alkyl,
R⁹ für Wasserstoff, C₁-C₆-alkyl, C₃-C₆-Alkenyl, C₃-C₆-Alkinyl, C₁-C₄-Alkoxy-C₁-C₄-alkoxy, Hydroxycarbonyl-C₁-C₄-alkyl, (C₁-C₆-Alkoxy)carbonyl-C₁-C₄-alkyl, (C₃-C₆-Alkenyloxy)carbonyl-C₁-C₄-alkyl, (C₃-C₆-Alkinyloxy)carbonyl-C₁-C₄-alkyl oder C₁-C₄-Alkoxy-(C₁-C₄-alkoxy)carbonyl-C₁-C₄-alkyl und
R¹⁰ für Wasserstoff, C₁-C₆-Alkyl, Hydroxycarbonyl oder (C₁-C₆-Alkoxy)carbonyl stehen,
sowie die landwirtschaftlich brauchbaren Salze der Verbindungen I.

2. Substituierte 2-Phenyl-3(2H)-pyridazinone der Formel I nach Anspruch 1 wobei die Variablen folgende Bedeutungen haben:
R¹ C₁-C₄-Alkyl;
R² Halogen;
R³ Halogen;
R⁴ Wasserstoff, Formyl, C₁-C₄-Alkyl, C₁-C₄-Halogenalkyl, -OR⁵, -CH=N-OR⁶, -CO-OR⁹ oder 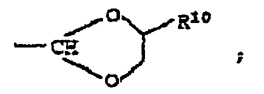
R⁵ C₁-C₆-Alkyl, C₃-C₆-Alkenyl, C₃-C₆-Alkinyl oder (C₁-C₆-Alkoxy)carboxyl-C₁-C₄-alkyl;
R⁶ Wasserstoff oder C₁-C₄-Alkyl;
R⁹ Wasserstoff, C₁-C₆-Alkyl, C₃-C₆-Alkenyl, C₃-C₆-Alkinyl, C₁-C₄-Alkoxy-C₁-C₄-alkoxy oder (C₁-C₆-Alkoxy)carbonyl-C₁-C₄-alkyl;
R¹⁰ Wasserstoff oder C₁-C₆-Alkyl.

3. Verwendung der substituierten 2-Phenyl-3(2H)-pyridazinone der Formel I und dar landwirtschaftlich brauchbaren Salze von I, gemäß Anspruch 1 oder 2, als Herbizide oder zur Desikkation und/oder Defoliation von Pflanzen.

4. Herbizides Mittel, enthaltend eine herbizid wirksame Menge mindestens eines substituierten 2-Phenyl-3(2H)-pyridazinons der Formel I oder eines landwirtschaftlich brauchbaren Salzes von I, gemäß Anspruch 1, und mindestens einen inerten flüssi-gen und/oder festen Trägerstoff sowie gewünschtenfalls mindestens einen oberflächenaktiven Stoff.

5. Mittel zur Desikkation und/oder Defoliation von Pflanzen, enthaltend eine desikkant und/oder defoliant wirksame Menge 10 mindestens eines substituierten 2-Phenyl-3(2H)-pyridazinons der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

6. Verfahren zur Herstellung von herbizid wirksamen Mitteln, **dadurch gekennzeichnet, daß** man eine herbizid wirksame Menge mindestens eines substituierten 2-Phenyl-3(2H)-pyridazinons der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

7. Verfahren zur Herstellung von desikkant und/oder defoliant wirksamen Mitteln, **dadurch gekennzeichnet, daß** man eine desikkant und/oder defoliant wirksame Menge mindestens eines substituierten 2-Phenyl-3(2H)-pyridazinons der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

8. Verfahren zur Bekämpfung von unerwünschtem Pflanzenwuchs, **dadurch gekennzeichnet, daß** man eine herbizid wirksame Menge mindestens eines substituierten 2-Phenyl-3(2H)-pyridazinons der Formel I oder eines landwirtschaftlich brauchbaren Salzes von I, gemäß Anspruch 1, auf Pflanzen, deren Lebensraum oder auf Saatgut einwirken läßt.

9. Verfahren zur Desikkation und/oder Defoliation von Pflanzen, **dadurch gekennzeichnet, daß** man eine desikkant und/oder defoliant wirksame Menge mindestens eines substituierten 2-Phenyl-3(2H)-pyridazinons der Formel I oder eines landwirtschaftlich brauchbaren Salzes von I, gemäß Anspruch 1, auf Pflanzen einwirken läßt.

10. Verfahren nach Anspruch 1, **dadurch gekennzeichnet, daß** man Baumwolle behandelt.

11. Verfahren zur Herstellung von substituierten 2-Phenyl-3(2H)-pyridazinonen der Formel I gemäß Anspruch 1, **dadurch gekennzeichnet, daß** man 4-Chlor-5-(halogen)alkylthio-2-phenyl-3(2H)-pyridazinone der Formel III 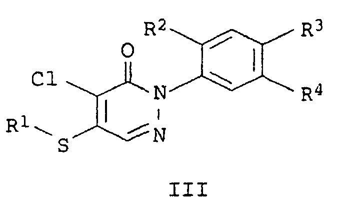 in einem inerten Lösungsmittel mit Ammoniak umsetzt oder zunächst am Schwefel oxidiert und die Oxidationsprodukte IV 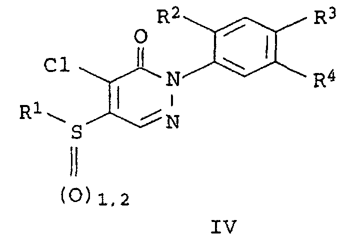 dann mit Ammoniak umsetzt.

## Claims

1. A substituted 2-phenyl-3(2H)-pyridazinone of the formula I 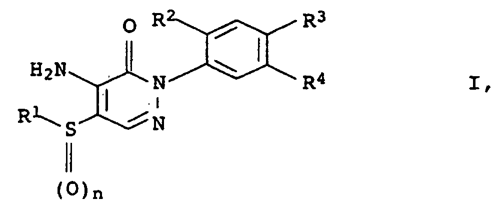 where
n is 1 or 2;
R¹ is C₁-C₄-alkyl or C₁-C₄-haloalkyl;
R² is hydrogen or halogen;
R³ is halogen or cyano;
R⁴ is hydrogen, nitro, cyano, formyl, hydroxylamino, C₁-C₄-alkyl, C₁-C₄-haloalkyl, -OR⁵, -CH=N-OR⁶, -CH=C(R⁷)-CO-OR⁸, -CO-OR⁹, (C₁-C₆-alkylsulfonyl)amino, di(C₁-C₆-alkylsulfonyl)amino or 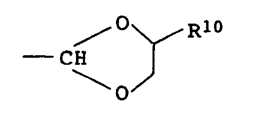 where
R⁵ is C₁-C₆-alkyl, C₃-C₆-alkenyl, C₃-C₆-alkynyl, (C₁-C₆-alkoxy)carbonyl-C₁-C₄-alkyl, (C₃-C₆-alkenyloxy)carbonyl-C₁-C₄-alkyl, (C₃-C₆-alkynyloxy)carbonyl-C₁-C₄-alkyl or C₁-C₄-alkoxy-(C₁-C₄-alkoxy)carbonyl-C₁-C₄-alkyl,
R⁶ is hydrogen, C₁-C₄-alkyl, hydroxycarbonyl-C₁-C₄-alkyl or (C₁-C₄-alkoxy)carbonyl-C₁-C₄-alkyl,
R⁷ is hydrogen, halogen or C₁-C₄-alkyl,
R⁸ is hydrogen or C₁-C₆-alkyl,
R⁹ is hydrogen, C₁-C₆-alkyl, C₃-C₆-alkenyl, C₃-C₆-alkynyl, C₁-C₄-alkoxy-C₁-C₄-alkoxy, hydroxycarbonyl-C₁-C₄-alkyl, (C₁-C₆-alkoxy)carbonyl-C₁-C₄-alkyl, (C₃-C₆-alkenyloxy)carbonyl-C₁-C₄-alkyl, (C₃-C₆-alkynyloxy)carbonyl-C₁-C₄-alkyl or C₁-C₄-alkoxy-(C₁-C₄-alkoxy)carbonyl-C₁-C₄-alkyl and
R¹⁰ is hydrogen, C₁-C₆-alkyl, hydroxycarbonyl or (C₁-C₆-alkoxy)carbonyl,
and the agriculturally useful salts of the compounds I.

2. A substituted 2-phenyl-3(2H)-pyridazinone of the formula I as claimed in claim 1, where:
R¹ is C₁-C₄-alkyl;
R² is halogen;
R³ is halogen;
R⁴ is hydrogen, formyl, C₁-C₄-alkyl, C₁-C₄-haloalkyl, -OR⁵, -CH=N-OR⁶, -CO-OR⁹ or 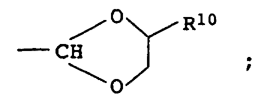
R⁵ is C₁-C₆-alkyl, C₃-C₆-alkenyl, C₃-C₆-alkynyl or (C₁-C₆-alkoxy)carbonyl-C₁-C₄-alkyl;
R⁶ is hydrogen or C₁-C₄-alkyl;
R⁹ is hydrogen, C₁-C₆-alkyl, C₃-C₆-alkenyl, C₃-C₆-alkynyl, C₁-C₄-alkoxy-C₁-C₄-alkoxy or (C₁-C₆-alkoxy)carbonyl-C₁-C₄-alkyl;
R¹⁰ is hydrogen or C₁-C₆-alkyl.

3. The use of the substituted 2-phenyl-3(2H)-pyridazinones of the formula I and the agriculturally useful salts of I, as claimed in claim 1 or 2, as herbicides or for the desiccation and/or defoliation of plants.

4. A herbicidal composition comprising a herbicidally effective amount of at least one substituted 2-phenyl-3(2H)-pyridazinone of the formula I or an agriculturally useful salt of I, as claimed in claim 1, and at least one inert liquid and/or solid carrier and, if desired, at least one surfactant.

5. A composition for the desiccation and/or defoliation of plants, comprising such an amount of at least one substituted 2-phenyl-3(2H)-pyridazinone of the formula I or an agriculturally useful salt of I, as claimed in claim 1, that it has desiccant and/or defoliant action, and at least one inert liquid and/or solid carrier and, if desired, at least one surfactant.

6. A process for the preparation of herbicidally active compositions, which comprises mixing a herbicidally effective amount of at least one substituted 2-phenyl-3(2H)-pyridazinone of the formula I or an agriculturally useful salt of I, as claimed in claim 1, and at least one inert liquid and/or solid carrier and, if desired, at least one surfactant.

7. A process for the preparation of compositions which have desiccant and/or defoliant action, which comprises mixing such an amount of at least one substituted 2-phenyl-3(2H)-pyridazinone of the formula I or an agriculturally useful salt of I, as claimed in claim 1, that it has desiccant and/or defoliant action, and at least one inert liquid and/or solid carrier and, if desired, at least one surfactant.

8. A method for controlling undesirable vegetation, which comprises allowing a herbicidally effective amount of at least one substituted 2-phenyl-3(2H)-pyridazinone of the formula I or an agriculturally useful salt of I, as claimed in claim 1, to act on plants, their habitat or on seeds.

9. A method for the desiccation and/or defoliation of plants, which comprises allowing such an amount of at least one substituted 2-phenyl-3(2H)-pyridazinone of the formula I or an agriculturally useful salt of I, as claimed in claim 1, to act on plants that it has desiccant and/or defoliant action.

10. A method as claimed in claim 10, wherein cotton is treated.

11. A process for preparing a substituted 2-phenyl-3(2H)-pyridazinone of the formula I as claimed in claim 1, which comprises reacting a 4-chloro-5-(halo)alkylthio-2-phenyl-3(2H)-pyridazinone of the formula III 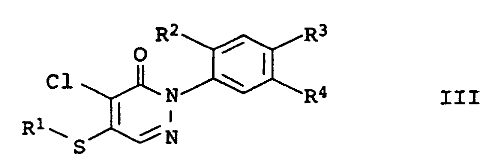 in an inert solvent with ammonia, or initially oxidizing it at the sulfur and then reacting the oxidation product IV 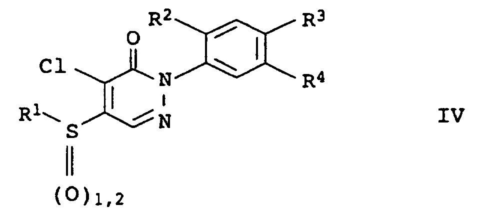 with ammonia.

## Revendications

1. 2-phényl-3(2H)-pyridazinones substituées de formule générale 1 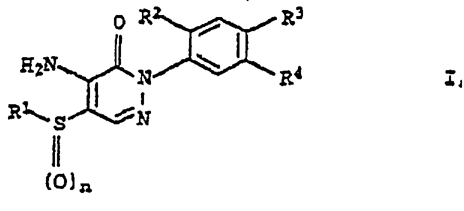 où les variables ont les significations suivantes:
n 1 ou 2;
R¹ alkyle en C₁-C₄ ou halogénoalkyle en C₁-C₄;
R² hydrogène ou halogéno;
R³ halogéno ou cyano;
R⁴ hydrogène, nitro, cyano, formyle, hydrpxylamino, alkyle en C₁-C₄, halogénoalkyle en C₁-C₄, -OR⁵, -CH=N-OR⁶, -CH=C(R⁷)-CO-OR⁸, -CO-OR⁹, (alkylsulfonyl en C₁-C₆)amino, di(alkylsulfonyl en C₁-C₆)amino ou 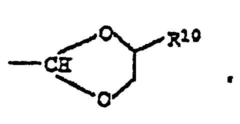 où
R⁵ désigne un groupe alkyle en C₁-C₆, alcényle en C₃-C₆, alcynyle en C₃-C₆, (alcoxy en C₁-C₆)carbonyl-alkyle(C₁-C₄), (alcényloxy en C₃-C₆)carbonyl-alkyle(C₁-C₄), (alcynyloxy en C₃-C₆)carbonyl-alkyle(C₁-C₄) ou alcoxy (C₁-C₄)-(alcoxy en C₁-C₄)carbonyl-alkyle(C₁-C₄),
R⁶ représente l'hydrogène ou un groupe alkyle en C₁-C₄, hydroxycarbonyl-alkyle(C₁-C₄) ou (alcoxy en C₁-C₄)carbonyl-alkyle(C₁-C₄),
R⁷ désigne l'hydrogène ou un groupe halogéno ou alkyle en C₁-C₄,
R⁸ représente l'hydrogène ou un groupe alkyle en C₁-C₆,
R⁹ représente l'hydrogène ou un groupe alkyle en C₁-C₆, alcényle en C₃-C₆, alcynyle en C₃-C₆, alcoxy(C₁-C₄)alcoxy(C₁-C₄), hydroxycarbonyl-alkyle(C₁-C₄), (alcoxy en C₁-C₆)carbonyl-alkyle(C₁-C₄), (alcényloxy en C₃-C₆)carbonyl-alkyle(C₁-C₄), (acynyloxy en C₃-C₆)carbonyl-alkyle(C₁-C₄) ou alcoxy en C₁-C₆-(alcoxy en C₁-C₄)carbonyl-alkyle(C₁-C₄) et
R¹⁰ représente l'hydrogène ou un groupe alkyle en C₁-C₆, hydroxycarbonyle ou (alcoxy en C₁-C₆)carbonyle,
ainsi que les sels des composés I utilisables en agriculture.

2. 2-phényl-3(2H)-pyridazinones substituées de formule I selon la revendication 1, où les variables ont les significations suivantes:
R¹ alkyle en C₁-C₄;
R² halogéno;
R³ halogéno;
R⁴ hydrogène, formyle, alkyle en C₁-C₄, halogénoalkyle en C₁-C₄, -OR⁵, -CH=N-OR⁶, -CO-OR⁹ ou 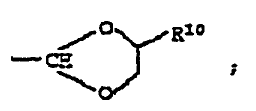
R⁵ alkyle en C₁-C₆, alcényle en C₃-C₆, alcynyle en C₃-C₆ ou (alcoxy en C₁-C₆)carbonyl-alkyle(C₁-C₄);
R⁶ hydrogène ou alkyle en C₁-C₄;
R⁹ hydrogène, alkyle en C₁-C₆, alcényle en C₃-C₆, alcynyle en C₃-C₆, alcoxy(C₁-C₄)-alcoxy(C₁-C₄) ou (alcoxy en C₁-C₆)carbonyl-alkyle(C₁-C₄);
R¹⁰ hydrogène ou alkyle en C₁-C₆.

3. Utilisation de 2-phényl-3-(2H)-pyridazinones substituées de formule I et des sels de I utilisables en agriculture, selon la revendication 1 ou 2, comme herbicides ou pour la dessiccation et/ou la défoliation des plantes.

4. Produit herbicide, contenant une quantité efficace du point de vue herbicide d'au moins une 2-phényl-3(2H)-pyridazinone substituée de formule I ou d'un sel de I utilisable en agriculture, selon la revendication 1, et au moins un support inerte liquide et/ou solide, ainsi qu'éventuellement au moins une substance tensioactive.

5. Produit pour la dessiccation et/ou la défoliation de plantes, contenant une quantité à efficacité dessiccante et/ou défoliante d'au moins une 2-phényl-3(2H)-pyridazinone substituée de formule I ou d'un sel de I utilisable en agriculture, selon la revendication 1, et au moins un support inerte liquide et/ou solide, ainsi qu'éventuellement au moins une substance tensioactive.

6. Procédé pour la préparation de produits à activité herbicide, **caractérisé par le fait qu'**on mélange une quantité à activité herbicide d'au moins une 2-phényl-3(2H)-pyridazinone substituée de formule I ou d'un sel de I utilisable en agriculture, selon la revendication 1, et au moins un support inerte liquide et/ou solide, ainsi qu'éventuellement au moins une substance tensioactive.

7. Procédé pour la préparation de produits à activité dessiccante et/ou défoliante, **caractérisé par le fait qu'**on mélange une quantité à activité dessiccante et/ou défoliante d'au moins une 2-phényl-3(2H)-pyridazinone substituée de formule I ou d'un sel de I utilisable en agriculture, selon la revendication 1, et au moins un support inerte liquide et/ou solide, ainsi qu'éventuellement au moins une substance tensioactive.

8. Procédé pour la lutte contre la croissance indésirable de plantes, **caractérisé par le fait qu'**on fait agir une quantité à efficacité herbicide d'au moins une 2-phényl-3(2H)-pyridazinone substituée de formule I ou d'un sel de I utilisable en agriculture, selon la revendication 1, sur des plantes, leur biotope ou sur des semences.

9. Procédé pour la dessiccation et/ou défoliation de plantes, **caractérisé par le fait qu'**on fait agir une quantité à efficacité dessiccante et/ou défoliante d'au moins une 2-phényl-3(2H)-pyridazinone substituée de formule I ou d'un sel de I utilisable en agriculture, selon la revendication 1, sur des plantes.

10. Procédé selon la revendication 9, **caractérisé par le fait qu'**on traite du coton.

11. Procédé pour la préparation de 2-phényl-3(2H)-pyridazinones substituées de formule I selon la revendication 1, **caractérisé par le fait qu'**on fait réagir de la 4-chloro-5-(halogéno)alkylthio-2-phényl-3(2H)-pyridazinone de formule III 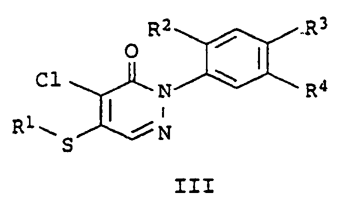 dans un solvant inerte avec de l'ammoniac, ou on oxyde ensuite sur le soufre et on fait ensuite réagir les produits d'oxydation IV 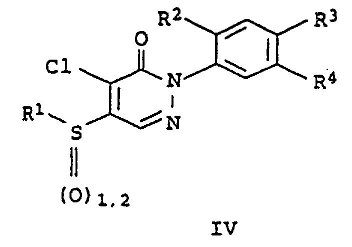 avec de l'ammoniac.
